Europäisches Patentamt

European Patent Office

Office européen des brevets

(11) Publication number: **0 219 204**
**A2**

(12) # EUROPEAN PATENT APPLICATION

(21) Application number: **86306309.5**

(22) Date of filing: **15.08.86**

(51) Int. Cl.⁴: **A 61 N 1/36**

(30) Priority: **15.08.85 GB 8520473**

(43) Date of publication of application:
**22.04.87 Bulletin 87/17**

(84) Designated Contracting States:
**GB IT NL**

(71) Applicant: **Grenfell, Hugh Wilmott**
**11 Maes-y-Bryn Morriston**
**Swansea West Glamorgan SA6 6HE(GB)**

(72) Inventor: **Grenfell, Hugh Wilmott**
**11 Maes-y-Bryn Morriston**
**Swansea West Glamorgan SA6 6HE(GB)**

(74) Representative: **Austin, Hedley William et al,**
**Urquhart-Dykes & Lord Alexandra House 1 Alexandra**
**Road**
**Swansea West Glamorgan SA1 5ED(GB)**

(54) **Electrostimulation of muscles.**

(57) Apparatus for the electrostimulation of leg and hip muscles e.g. if paralysed, comprises a harness arrangement to be worn so as to place electrodes (50,51;65,66) of the harness in contact with appropriate parts of the body for stimulating at least the quadriceps and gluteals, and electronic apparatus for providing electric currents to the electrodes.

This electronic apparatus includes circuits which are electrically isolated from each other to drive the electrodes (50,51;65,66) for the quadriceps and gluteals respectively: the quadriceps and gluteals of each leg can be stimulated without causing any unwanted stimulation of the interposed psoas.

EP 0 219 204 A2

FIG. 4

FIG 5

## ELECTROSTIMULATION OF MUSCLES

This invention relates to the electrostimulation of muscles, for example paralysed muscles of an at least partially paralysed patient, to promote movement for example walking, standing or sitting.

It has been well known for many years that muscles stimulated with electricity can be caused to contract and this subject is well documented. My U.K. patent specification No. 2 136 297 describes equipment for stimulating the legs and hips of a paralysed person to produce a smooth contraction of the muscles to enable that person to walk.

However, patients who are paralysed above a certain level require the use of postural muscles to enable them to balance and to maintain an erect posture. Since the body itself is a conductor of electricity it has proved extremely difficult to avoid the stimulation of one muscle group interfering with the stimulation of another muscle group whether the muscles are adjacent each other or remote. In fact, the further apart the muscles are, the more complex the interference is. Also, if the muscles require substantially different levels of stimulation, then a potential difference is set up through the body which may stimulate any intervening muscles between the two stimulated muscle groups. For example, it is required to stimulate the quadriceps for extension of the knee and the gluteals for extending the hip. Interposing is the

- 2 -                                              0219204

illiopsoas muscle which flexes the hip. Since the illiopsoas (or psoas) is an extremely strong muscle it picks up the stimulation between the quadriceps muscle and the gluteals and flexes the hip as well as reducing the stimulation to the quadriceps, causing the knee to give way and the patient to fall to the ground.

I have now devised techniques for electrostimulation of the desired muscles, which overcome the above problems.

In accordance with this invention, there is provided an electrode harness arranged to be worn so as to place electrodes of the harness in contact with appropriate parts of the body for stimulating the quadriceps and gluteals, and electronic apparatus for providing electric currents to the electrodes to stimulate the quadriceps and gluteals, said electronic apparatus including circuits which are electrically isolated from each other and include the electrodes respectively for the quadriceps and gluteals.

With this arrangement, the quadriceps and gluteals are stimulated as required without any unwanted stimulation of the psoas also occuring.

Also in accordance with this invention, there is provided a method of electrostimulating the quadriceps and gluteals of a patient, comprising supplying electrical currents to electrodes appropriately placed in contact with the body, these currents being supplied over respective circuits which are electrically isolated from each other so that unwanted stimulation of the psoas is avoided.

An embodiment of this invention will now be described by way of example only and with reference to the accompanying drawings, in which:

Figure 1 is a diagrammatic representation of the lower part of the human body;

Figure 2 is a circuit diagram of the equipment;

Figure 3 is a diagram to illustrate the distribution of electric pulses to the muscles;

Figure 4 shows a harness for one leg; and

Figure 5 shows an electrode belt.

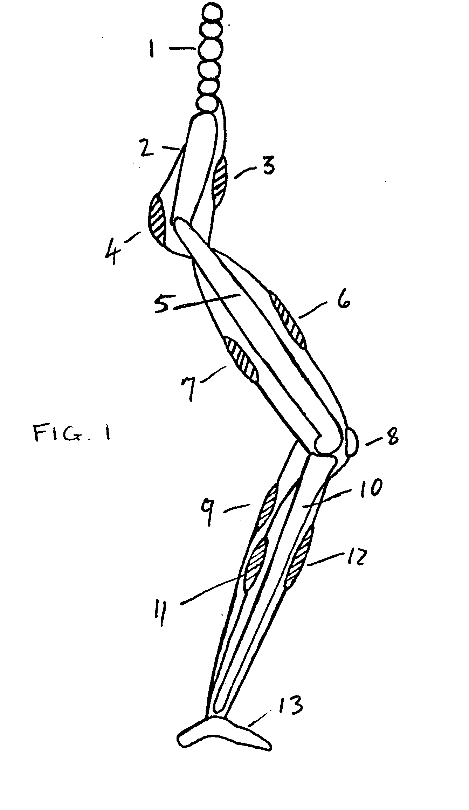

Referring to Figure 1, there is shown the spinal column 1 and pelvis 2 of a patient. One leg and its muscle system is shown, namely the psoas muscle 3 attached to the spinal column and the top of the femur and is used for flexing the hip, the gluteal muscle group 4 which is attached to the ileac crest at the top of the pelvis and the back of the femur 5 and serves to extend the hips, the quadriceps muscle group 6 which is attached from the top of the femur through the patella 8 to the tibia and is used for extending the knee, the hamstring group 7 attached at the back of the femur and the back of the tibia and used for flexing the knee, the gastronemius attached to the femur and to the heel, and the soleus 11 attached to the tibia and the heel: the gastronemius and the soleus are both part of the plantaflexor group and are used for extending the ankle. The dosiflexors 12 are also shown and are attached to the tibia and foot and serve for flexing the ankle.

In order to enable a patient to stand it is necessary especially in the higher lesions to stimulate the gluteals, the quadriceps and the plantaflexors. In accordance with this invention, the gluteals and quadriceps are stimulated over circuits which are isolated from each other, to avoid a short circuiting of the stimulation pulses through the psoas (which, being strong, would react immediately and cause the hips to flex instead of extend), and a reduction of power to the quadriceps and collapse of the knee.

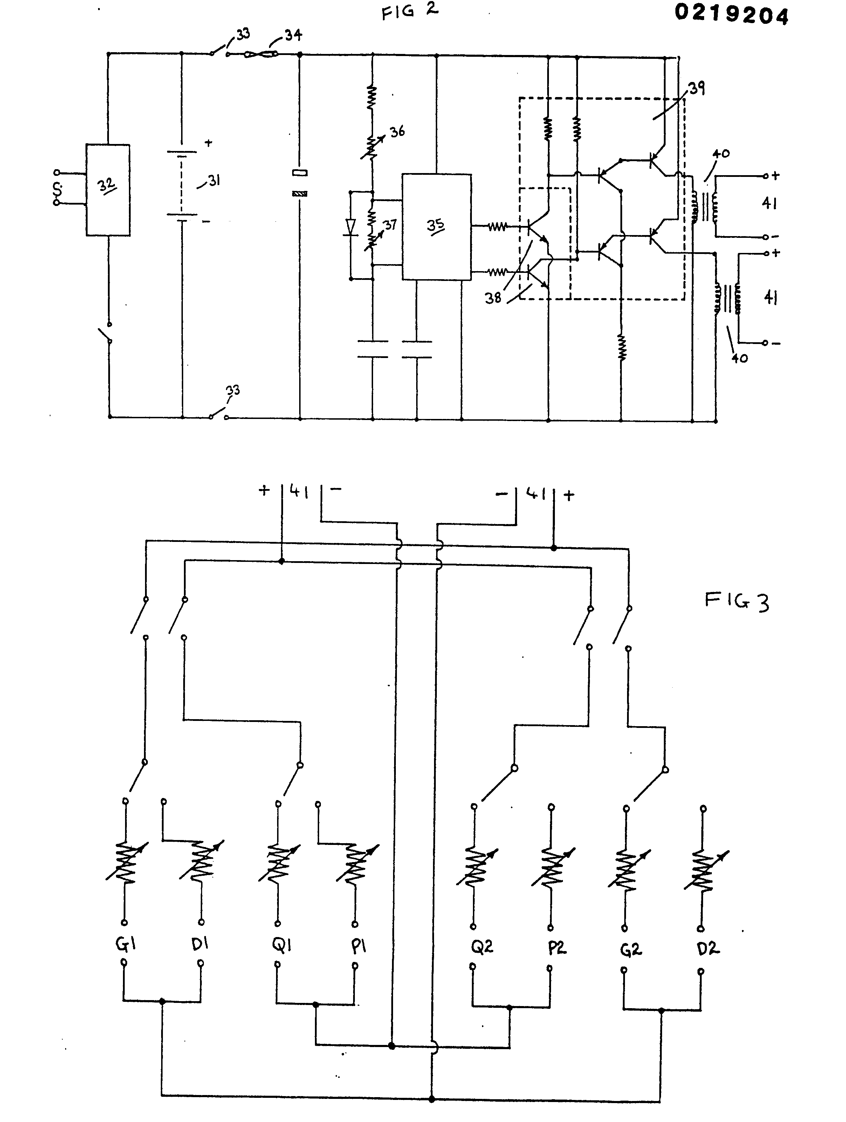

Figure 2 shows in particular the pulse generator employed, This includes a battery 31 (usually 12 volts) and a battery charger 32 powered from the mains. The battery supplies an integrated circuit 35 (which generates the basic stimulation pulses) via an ON/OFF switch 33 and a fuse 34. The pulses from circuit 35 are adjustable for pulse width and pulse rate by potentiometers 36,37 between the ranges 0.2 and 1.8 milliseconds and 10 and 40 Hertz respectively. The pulses from circuit 35 drive an output stage integrated circuit 38, which in turn drives Darlington-configured transistors 39. These drive transformers 40, providing two separate and isolated outputs 41, one for the posture and one for the leg muscles.

Figure 3 shows the distribution of pulses from the two isolated outputs 41 to the various electrodes. For one leg, there are electrode pairs G1, D1, P1 and Q1 serving the gluteals, dorsiflexors, psoas and quadriceps, respectively of that leg: for the other leg, there are corresponding electrode pairs G2, D2, P2 and Q2. For each leg the gluteals and dorsiflexors electrodes receive their pulses from one of the outputs 41, and the quadriceps and psoas receive their pulses from the other output 41. The pulses are distributed to the muscles through respective emitter-follower circuits, shown diagrammatically, with individual potentiometers providing control.

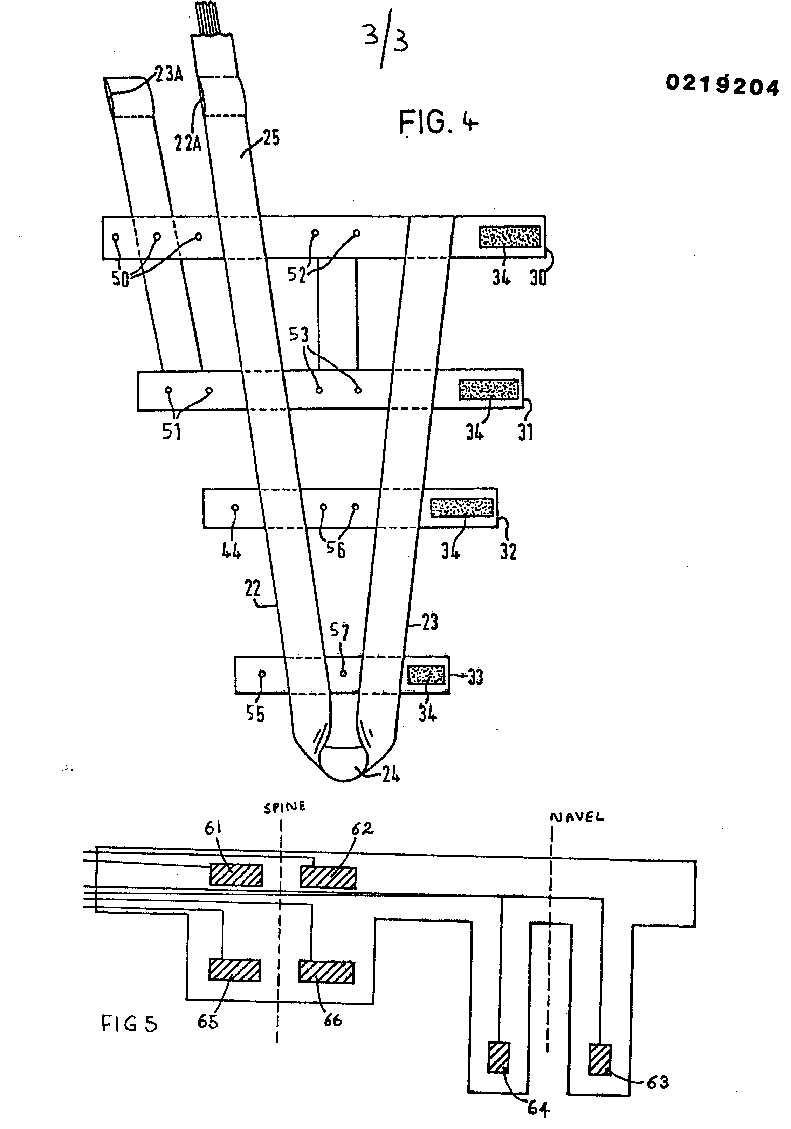

Figure 4 shows a harness assembly for one leg. It comprises bands 30-33 carrying electrodes 50-57 and connected together by two straps 22, 23 running the length of the leg, with a strap portion 24 to pass under the instep. The bands are provided with fastening means 34 (e.g. "Velcro") so that they can be secured around the leg and place the electrodes in contact against the body in the correct positions. Wires to the electrodes are sewn into the straps 22, 23 and the electrodes are shown as follows: 50 and 51 for the quadriceps, 52 and 53 for the hamstring muscles, 54and 55 for the dorsiflexors, and 56 and 57 for the plantaflexors. This harness is as more fully described in my U.K. patent specification No. 2 136 297. Figure 5 further shows an electrode belt to be worn with a pair of the leg harnesses. This has electrodes 61,62 for the right and left psoas and the belt is worn so that these are positioned at the back, either side of the spine. Smaller electrodes 63,64 for the right and left psoas are provided to locate on the abdomen either side and slightly lower than the navel. The gluteals are stimulated by electrodes 65,66 on the belt, placed on the back either side of the spine and just below the iliac crest, and paired with the electrodes 52 at the top of the leg harnesses.

## CLAIMS

1.        Electrostimulation apparatus comprising an electrode harness arranged to be worn so as to place electrodes of the harness in contact with appropriate parts of the body for stimulating predetermined leg and hip muscles, and electronic apparatus for providing electric currents to the electrodes to stimulate those muscles, characterised in that the harness includes electrodes (50, 51; 65, 66) positioned for contacting appropriate parts of the body for stimulating the quadriceps and gluteals, and the electronic apparatus includes circuits (40, 41; 40, 41) which are electrically isolated from each other and which include the electrodes respectively for the quadriceps and gluteals.

2.        Electrostimulation apparatus as claimed in claim 1, characterised in that the electronic apparatus comprises one said circuit (40,41) which includes the electrodes for the quadriceps of one leg and the gluteals of the other leg, and another said circuit (isolated from the one circuit) which includes the electrodes for the gluteals of the one leg and the quadriceps of the other leg.

3.        Electrostimulation apparatus as claimed in claim 1 or 2, characterised in that the harness further includes electrodes (61-64) for stimulating the psoas which electrodes are connected into the same circuit as the electrodes (50,51) for stimulating the quadriceps.

4.        Electrostimulation apparatus as claimed in claim 1,2 or 3, characterised in that the harness further includes electrodes (54,55) for stimulating the dorsiflexors which electrodes are connected into the same circuit as the electrodes (65,66) for stimulating the gluteals.

5.        Electrostimulation apparatus as claimed in any preceding claim, characterised in that the electronic apparatus comprises a pulse generator (35-39) having an output stage driving two transformers (40,40) which provide two separate and isolated outputs (41,41).

6.        Electrostimulation apparatus as claimed in any preceding claim, characterised in that the harness comprises separate parts for the two legs and supporting the electrodes (50,51) for the quadriceps, and a third part for the hips and supporting the electrodes (65,66) for the gluteals.

7.        Electrostimulation apparatus as claimed in claim 6, in which said third part of the harness further supports electrodes (61-64) for the psoas, which electrodes include (for each leg) an electrode (61) for positioning on the back to the appropriate side of the spine and an electrode (63) for positioning on the abdomen to the appropriate side of and slightly lower than the navel.

FIG. 1

FIG 2

0219204

FIG 3

FIG. 4

FIG 5